# EUROPÄISCHE PATENTSCHRIFT

(11) **EP 0 789 745 B1**
(45) Veröffentlichungstag und Bekanntmachung des Hinweises auf die Patenterteilung: **24.03.1999**
(21) Anmeldenummer: 95937828.2
(22) Anmeldetag: 24.10.1995
(51) Int. Cl.: C11D 1/835, D06M 13/463, A61K 7/50

(54) **AVIVAGEMITTEL MIT VERBESSERTEM WIEDERBENETZUNGSVERMÖGEN**
SOFTENING AGENTS HAVING IMPROVED RE-WETTING ABILITY
AGENTS D'AVIVAGE PRESENTANT UN POUVOIR REMOUILLANT AMELIORE

(30) Priorität: 02.11.1994 DE 4439076
(43) Veröffentlichungstag der Anmeldung: 20.08.1997
(73) Patentinhaber: Henkel Kommanditgesellschaft auf Aktien, 40191 Düsseldorf (DE)
(72) Erfinder: BIGORRA LLOSAS, Joaquim, E-08203 Sabadell (ES); BRAU BALAQUE, Emili, E-43570 Santa Barabara (ES); PI SUBIRANA, Rafael, E-08400 Granollers (ES); SOLER CODINA, Antoni, E-08221 Terassa (ES)
(86) Internationale Anmeldenummer: EP9504157
(87) Internationale Veröffentlichungsnummer: WO9614379

(56) Entgegenhaltungen:
- DE-A- 4 232 448
- DE-C- 4 243 547

## Beschreibung

### Gebiet der Erfindung

Die Erfindung betrifft neue Avivagemittel mit einem Gehalt an phosphatierten Esterquats und nichtionischen Tensiden.

### Stand der Technik

Quaternierte Fettsäuretriethanolaminestersalze, sogenannte "Esterquats", haben in den letzten Jahren als ökotoxikologisch unbedenkliche Rohstoffe für Wäscheweichspüler zunehmend an Bedeutung gewonnen [vgl. O.Ponsati in **C.R. CED-Kongress, Barcelona, 167 (1992)** und R.Puchta in **C.R. CED-Kongress, Sitges, 59 (1993)**]. Zur Herstellung der Esterquats geht man üblicherweise von Fettsäurealkanolaminestem aus, die in Gegenwart von Isopropylalkohol mit geeigneten Alkylierungsmitteln wie beispiels weise Dimethylsulfat quatemiert werden. Dabei resultieren Konzentrate mit etwa 90 Gew.-% Feststoffgehalt im organischen Lösungsmittel, die mit Wasser auf die gewünschte Anwendungskonzentration verdünnt werden können. Die konzentrierten Produkte weisen zwar ausgezeichnete avivierende Eigenschaften auf, das Wiederbentzungsvermögen ist jedoch nicht optimal. Aus der **DE-C 4243547** (Henkel/Pulcra) sind phosphatierte Esterquats bekannt, die die Strukturmerkmale von kationischen (Esterquats) und anionischen Tensiden (Alkylphosphaten) aufweisen. Die Stoffe zeigen avivierende Eigenschaften und eignen sich zur Herstellung von Spinnfaserpräparationen. Das Avivagevermögen ist jedoch nicht sehr ausgeprägt, die Wiederbenetzung von mit diesen Mitteln behandeltem Gewebe ebenfalls wenig zufriedenstellend. Des weiteren sei auf die **DE-A 4232448** verwiesen, die Avivagemittel aus ethoxylierten Alkoholen und quartären Ammoniumsalzen mit Estergruppen beschreibt, bei denen die kationischen verbindungen als Sulfatsalze vorliegen.

Die Aufgabe der Erfindung hat nun darin bestanden, neue Avivagemittel zur Verfügung zu stellen, die Textilien oder Haaren einen guten Weichgriff und gleichzeitig eine verbesserte Wiederbentzbarkeit verleihen.

### Beschreibung der Erfindung

Gegenstand der Erfindung sind Avivagemittel mit verbessertem Wiederbenetzungsvermögen, enthaltend
(a) phosphatierte Esterquats der Formel **(I),** 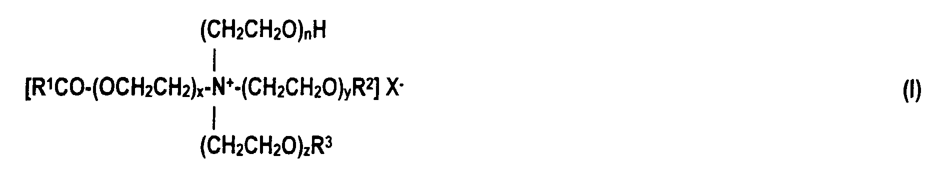 in der R¹CO für einen Acylrest mit 6 bis 22 Kohlenstoffatomen, R² und R3 unabhängig voneinander für Wasserstoff oder R¹CO, n für Zahlen von 1 bis 10, x, y und z für 1 oder Zahlen von 2 bis 30 und X für ein Alkyl- und/oder Dialkylphosphat mit 6 bis 22 Kohlenstoffatomen steht, und
(b) Alkoholpolyglycolether der Formel **(II)**,

   **R**^{**4**}**O(CH**_{**2**}**CH**_{**2**}**O)**_{**p**}**H (II)**

   in der R⁴ für Alkyl- und/oder Alkenylreste mit 6 bis 22 Kohlenstoffatomen und p für Zahlen von 1 bis 20 steht.

Überraschenderweise wurde gefunden, daß Abmischungen von phosphatierten Esterquats mit nichtionischen Tensiden vom Typ der Alkylpolyethylenglycolether ein verbessertes Wiederbenetzungsvermögen aufweisen.

### Phosphatierte Esterquats

Phosphatierte Esterquats stellen bekannte kationische Tenside dar, die nach der einschlägigen Methoden der präparativen organischen Chemie erhalten werden können. Ein Verfahren zu ihrer Herstellung gemäß der eingangs bereits genannten DE-C 4243547, auf deren Lehre hiermit ausdrücklich Bezug genommen wird, besteht dann, Fettsäurealkanolaminester in Gegenwart langkettiger Phosphorsäureester mit Ethylenoxid zu quatemieren. Typische Beispiele für phosphatierte Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden technische C_{12/18}-Kokosfettsäuren und insbesondere teilgehärtete C_{16/18}-Talg-bzw. Palmfettsäuren sowie elaidinsäurereiche C_{16/18}-Fettsäureschnitte eingesetzt. Zur Herstellung der quaternierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestem mit einem durchschnittlichen Veresterungsgrad von 1,5 bis 1,9 dar und leiten sich von technischer C_{16/18}-Talg- bzw. Palmfettsäure (Iodzahl 0 bis 40) ab. Die Quaternierung kann durch Umsetzung von 1 Mol Fettsäurealkanolaminester mit durchschnittlich 1 bis 10 und vorzugsweise 1 bis 5 Mol Ethylenoxid erfolgen. Die phosphatierten Esterquats stellen keine echten Phosphatierungsprodukte dar, vielmehr dient das eingesetzte langkettige Phosphat als Anion zum positiv geladenen Esterquat; es handelt sich hierbei also um einen Anion-Kation-Komplex. Als geeignete Phosphate, in deren Gegenwart die Quaternierung mit Ethylenoxid durchgeführt wird, kommen Mono- und/oder Dialkylester der Phosphorsäure bzw. technische Gemische in Betracht, bei denen sich die Alkylreste von Alkoholen mit 6 bis 22 und vorzugsweise 8 bis 12 Kohlenstoffatomen ableiten. Das Mono/Di-Verhältnis in den Phosphorsäureestern liegt vorzugsweise im Bereich von 0,5 : 1 bis 1 : 1 und vorzugsweise um den Wert 0,7 : 1. Besonders bevorzugt ist der Einsatz von Octyl/decyl- oder Laurylphosphaten mit einem Verhältnis von Mono/Diester von etwa 0,7 : 1. Das bevorzugte molare Einsatzverhältnis von Esterquat zu Alkyl- und/oder Dialkylphosphat beträgt ferner 0,5 : 1 bis 2 : 1 und vorzugsweise 1 : 1 bis 1,5 : 1. In in Summe besonders bevorzugt sind phosphatierte Esterquats der Formel **(I)**, in der R¹CO für einen Acylrest mit 12 bis 18 Kohlenstoffatomen und X für ein Alkyl- und/oder Dialkylphosphat mit 8 bis 12 Kohlenstoffatomen steht.

### Alkoholpolyglycolether

Alkoholpolyglycolether stellen bekannte nichtionische Tenside dar, die durch basenkatalysierte Anlagerung von Alkylenoxiden, vorzugsweise Ethylenoxid an primäre lineare oder verzweigte Alkohole erhalten werden können. Im Sinne der Erfindung kommen Ethylenoxid-Addukte an Fettalkohole und Oxoalkohole in Betracht. Typische Beispiele sind Anlagerungsprodukte von durchschnittlich 1 bis 20, vorzugsweise 5 bis 10 Mol Ethylenoxid an Capronalkohol, Caprylalkohol, 2-Ethylhexylalkohol, Caprinalkohol, Laurylalkohol, Iso-dodecylalkohol, Isotfidecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol und Erucylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Die Additionsprodukte können dabei eine konventionelle oder eingeengte Homologenverteilung aufweisen. Vorzugsweise werden Alkylpolyglycolether der Formel **(II)** eingesetzt, in der R⁴ für einen Alkylrest mit 6 bis 18 Kohlenstoffatomen und p für Zahlen von 5 bis 10 steht. Typische Beispiele sind die Anlagerungsprodukte von 5 bis 10 Mol Ethylenoxid an C_{8/10}-Vorlauffettalkohole oder technische Kokosfettalkoholschnitte. Die erfindungsgemäßen Mittel können die Komponenten (a) und (b) im Gewichtsverhältnis 80 : 20 bis 99 : 1 und vorzugsweise 92 : 8 bis 97 : 3 enthalten. Die Vermischung kann beispielsweise in einem Rührbehälter bei gegebenenfalls leicht erhöhten Temperaturen von etwa 40°C erfolgen. Hierbei handelt es sich um einen rein mechanischen Vorgang, eine chemische Reaktion findet nicht statt.

### Gewerbliche Anwendbarkeit

Die erfindungsgemäßen Avivagemittel weisen ein vorteilhaftes Wiederbenetzungsvermögen auf und verleihen Textilien und Haaren einen hervorragenden Weichgriff. Üblicherweise können sie in Form von Konzentraten in den Handel gelangen, die dann vom Verbraucher auf die Anwendungskonzentration verdünnt werden. Verdünnte wäßrige Lösungen kommen als Anbietungsformen demnach ebenfalls in Betracht. Die Mittel dienen der Avivage von Textilien, Fasern und Haaren und können daher weitere typische lnhaltsstoffe, insbesondere auch weitere kompatible Tenside in untergeordneten Mengen enthalten. Synergistische Effekte werden beispielsweise in Kombination der erfindungsgemäßen Mittel mit Alkyl- und/oder Alkenylsulfaten und/oder soil repellant-Polymeren (z.B. Repel-o-tex, Rhône-Poulenc) beobachtet.

### Beispiele

### Allgemeine Herstellungsvorschrift

**Veresterung.** In einem 1-I-Dreihalskolben mit Rührer, Innenthemometer und Destillationsaufsatz wurden 1,9 Mol teilgehärtete Palmfettsäure, 1 mol Triethanolamin und 1,4 g 50 Gew.-%ige unterphosphorige Säure gegeben. Über einen Zeitraum von 4 h wurde die Reaktionsmischung bei einem verminderten Druck von 40 mbar auf eine Temperatur von 160°C erhitzt, bis die Säurezahl unterhalb von 5 lag. Anschließend wurde der rohe Fettsäuretriethanolaminester abgekühlt, der Reaktionsansatz entspannt und unter ständigem Rühren innerhalb von 15 min 1 Liter Luft durchgeleitet.

**Quaternierung.** In einem 1-I-Stahlautoklaven wurden 1 mol des technischen Veresterungsproduktes vorgelegt und mit (1) 1 Mol Phosphorsäureoctyl/decylester (Mono/Di-Verhältnis = 0,7) bzw. (Produkt "A") bzw. (2) 0,66 Mol Phosphorsäurelaurylester (Mono/Di-Verhältnis = 0,7) (Produkt "B") versetzt. Anschließend wurden innerhalb von 2 h 1 Mol Ethylenoxid bei einem Druck von 3 bar und einer Temperatur von 105°C aufgepreßt. Produkt "C" stellt ein Esterquat analog "A", dar welches jedoch mit Methylsulfat quaterniert worden ist (Dehyquart® Au 46).

### Anwendungstechnische Beispiele

Durch wiederholtes Waschen gehärtetes Baumwollgewebe (Molton) wurde unter Anwendung des Foulardverfahrens mit den Produkten der Beispiele 1 bis 3 sowie der Vergleichsbeispiele V1 bis V3 behandelt. Dabei galten folgende Vorgaben: Konzentration : 30 g/l Avivagemittel, Flottenaufnahme : ca. 80 Gew.-% bezogen auf trockenes Gewebe, Trocknung : 3 min bei 180°C. Von den ausgerüsteten Prüfgeweben wurden die Wiederbenetzbarkeit und der Weichgriff beurteilt. Zur Prüfung der Wiederbenetzbarkeit wurde 1 Tropfen Wasser aus einer Höhe von 4 cm auf die Textiloberfläche getropft und die Zeit bestimmt, bis der Tropfen vom Gewebe aufgesaugt worden war. Die Beurteilung des Weichgriffs erfolgte subjektiv durch 6 erfahrene Personen, die auf einer Skala von 0 = hart und rauh bis 6 = weich und voluminös Noten vergeben konnten. Die Testergebnisse sind in Tabelle 1 zusammengefaßt:

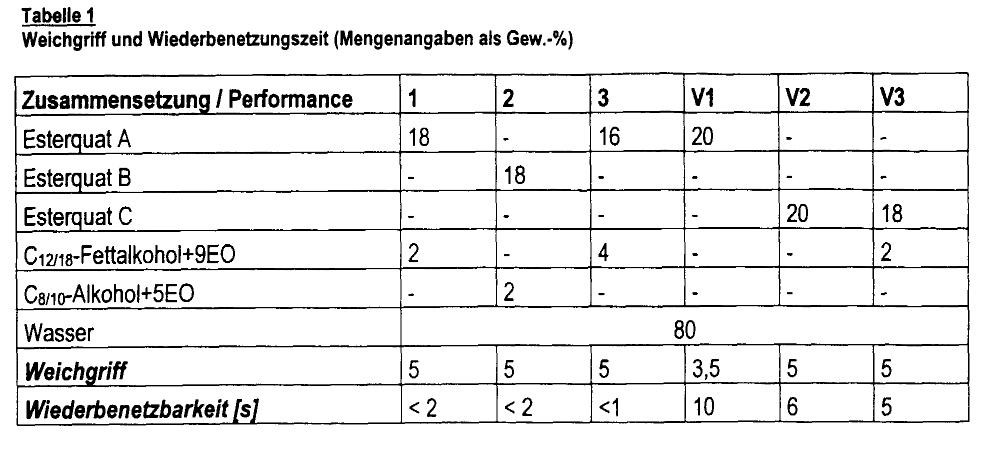

## Patentansprüche

1. Avivagemittel mit verbessertem Wiederbenetzungsvermögen, enthaltend
(a) phosphatierte Esterquats der Formel **(I)**, 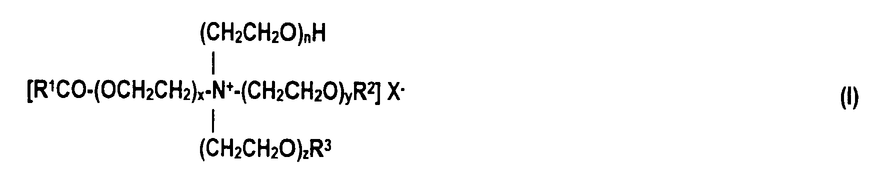 in der R¹CO für einen Acylrest mit 6 bis 22 Kohlenstoffatomen, R² und R3 unabhängig voneinander für Wasserstoff oder R¹CO, n für Zahlen von 1 bis 10, x, y und z für 1 oder Zahlen von 2 bis 30 und X für ein Alkyl- und/oder Dialkylphosphat mit 6 bis 22 Kohlenstoffatomen steht, und
(b) Alkoholpolyglycolether der Formel **(II)**,
**R**^{**4**}**O(CH**_{**2**}**CH**_{**2**}**O)**_{**p**}**H (II)**
in der R⁴ für Alkyl- und/oder Alkenylreste mit 6 bis 22 Kohlenstoffatomen und p für Zahlen von 1 bis 20 steht.

2. Mittel nach Anspruch 1, **dadurch gekennzeichnet**, daß sie phosphatierte Esterquats nach Formel **(I)** enthalten, in der R¹CO für einen Acylrest mit 12 bis 18 Kohlenstoffatomen und X für ein Alkyl- und/oder Dialkylphosphat mit 8 bis 12 Kohlenstoffatomen steht.

3. Mittel nach den Ansprüchen 1 und 2, **dadurch gekennzeichnet**, daß sie phosphatierte Esterquats enthalten, bei denen auf 1 Mol Alkyl- und/oder Dialkylphosphat 0,5 bis 2 Mol Fettsäurealkanolaminester entfallen.

4. Mittel nach den Ansprüchen 1 bis 3, **dadurch gekennzeichnet,** daß sie Alkylpolyglycolether der Formel **(II)** enthalten, in der R⁴ für einen Alkylrest mit 6 bis 18 Kohlenstoffatomen und p für Zahlen von 5 bis 10 steht.

5. Mittel nach den Ansprüchen 1 bis 4, **dadurch gekennzeichnet,** daß sie die Komponenten (a) und (b) im Gewichtsverhältnis 80 : 20 bis 99 : 1 enthalten.

## Claims

1. Softeners with improved rewetting behavior containing
(a) phosphated esterquats corresponding to formula (I): 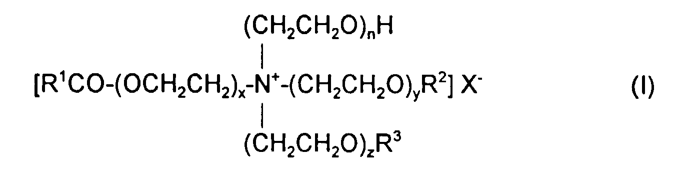 in which R¹CO is an acyl group containing 6 to 22 carbon atoms, R² and R³ independently of one another represent hydrogen or have the same meaning as R¹CO, n is a number of 1 to 10, x, y and z stand for 1 or for numbers of 2 to 30 and X is an alkyl and/or dialkyl phosphate containing 6 to 22 carbon atoms and
(b) alcohol polyglycol ethers corresponding to formula (II):
R⁴O(CH₂CH₂O)ₚH (II)
in which R⁴ is an alkyl and/or alkenyl group containing 6 to 22 carbon atoms and p is a number of 1 to 20.

2. Softeners as claimed in claim 1, characterized in that they contain phosphated esterquats corresponding to formula (I) in which R¹CO is an acyl group containing 12 to 18 carbon atoms and X is an alkyl and/or dialkyl phosphate containing 8 to 12 carbon atoms.

3. Softeners as claimed in claims 1 and 2, characterized in that they contain phosphated esterquats in which there are 0.5 to 2 moles of fatty acid alkanolamine esters to 1 mole of alkyl and/or dialkyl phosphate.

4. Softeners as claimed in claims 1 to 3, characterized in that they contain alkyl polyglycol ethers corresponding to formula **(II)** in which R⁴ is an alkyl group containing 6 to 18 carbon atoms and p is a number of 5 to 10.

5. Softeners as claimed in claims 1 to 4, characterized in that they contain components (a) and (b) n a ratio by weight of 80:20 to 99:1.

## Revendications

1. Agents d'avivage à pouvoir remouillant amélioré, renfermant
(a) des esters quaternisés phosphatés de la formule (I), 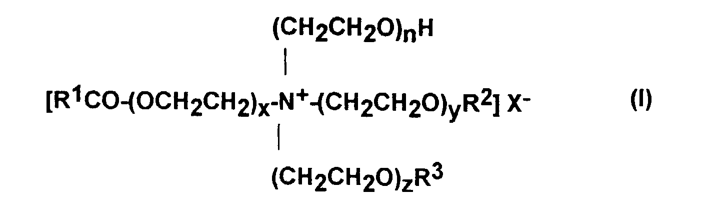 dans laquelle R¹CO représente un radical acyle comportant 6 à 22 atomes de carbone, R² et R³ correspondent indépendamment l'un de l'autre à l'hydrogène ou à R¹CO, n est égal à un nombre de 1 à 10, x, y et z représentent 1 ou un nombre de 2 à 30, et X correspond à un alkyl- et/ou à un dialkylphosphate comportant 6 à 22 atomes de carbone, et
(b) des polyglycoléthers d'alcools de la formule (II),
**R**^{**4**}**O(CH**_{**2**}**CH**_{**2**}**O)**_{**p**}**H (II)**
dans laquelle R⁴ représente un radical alkyle et/ou alcényle comportant 6 à 22 atomes de carbone et p correspond à un nombre de 1 à 20.

2. Agents selon la revendication 1, **caractérisés en ce qu**'ils renferment des esters quaternisés phosphatés selon la formule (I), dans laquelle R¹CO représente un radical acyle comportant 12 à 18 atomes de carbone et X correspond à un alkyl- et/ou à un dialkylphosphate comportant 8 à 12 atomes de carbone.

3. Agents selon les revendications 1 et 2, **caractérisés en ce qu'**ils renferment des esters quaternisés phosphatés, dans lesquels il y a 0,5 à 2 mol d'ester d'alcanolamine d'acide gras pour 1 mol d'alkyl- et/ou de dialkylphosphate.

4. Agents selon les revendications 1 à 3, **caractérisés en ce qu**'ils renferment des alkylpolyglycoléthers de la formule (II), dans laquelle R⁴ représente un radical alkyle comportant 6 à 18 atomes de carbone et p correspond à un nombre de 5 à 10.

5. Agents selon les revendications 1 à 4, **caractérisés en ce qu**'ils renferment les composants (a) et (b) dans un rapport pondéral de 80:20 à 99:1.
